# EUROPÄISCHE PATENTSCHRIFT

(11) **EP 1 472 263 B1**
(45) Veröffentlichungstag und Bekanntmachung des Hinweises auf die Patenterteilung: **30.08.2006**
(21) Anmeldenummer: 02803009.6
(22) Anmeldetag: 12.11.2002
(51) Int. Cl.: C07F 7/08, A61K 8/18, C11D 7/22, C11D 7/50

(54) **VERWENDUNG VON SILOXANEN ALS VERDAMPFBARE TRÄGER**
USE OF SILOXANES AS EVAPORABLE SUPPORTS
UTILISATION DE SILOXANES COMME SUPPORTS EVAPORABLES

(30) Priorität: 13.11.2001 DE 10155512
(43) Veröffentlichungstag der Anmeldung: 03.11.2004
(73) Patentinhaber: GE Bayer Silicones GmbH & Co. KG, 51368 Leverkusen (DE)
(72) Erfinder: EVERSHEIM, Hubertus, 42929 Wermelskirchen (DE); KROPFGANS, Martin, 51519 Odenthal (DE); NIENSTEDT, Sabine, 53155 Bonn (DE); LANGE, Horst, 44879 Bochum (DE)
(86) Internationale Anmeldenummer: PCT/EP2002/012596
(87) Internationale Veröffentlichungsnummer: WO 2003/042221

(56) Entgegenhaltungen:
- DATABASE CA [Online] CHEMICAL ABSTRACTS SERVICE, COLUMBUS, OHIO, US; SUGAI, MICHIHIRO ET AL: "Cleaning agents containing linear or branched polyorganosiloxanes with specified Kauri Butanol value and having no ozone layer depleting properties" retrieved from STN Database accession no. 133:106626 CA XP002230367 & JP 2000 204398 A (SHIN-ETSU CHEMICAL INDUSTRY CO., LTD., JAPAN) 25. Juli 2000 (2000-07-25)
- DATABASE CA [Online] CHEMICAL ABSTRACTS SERVICE, COLUMBUS, OHIO, US; KUMAGAI, SHIGENORI ET AL: "Cosmetic compositions containing disiloxanes" retrieved from STN Database accession no. 93:137891 CA XP002230368 & JP 55 020737 A (SHISEIDO CO., LTD., JAPAN) 14. Februar 1980 (1980-02-14)
- KOPYLOV V M ET AL: "FEATURES OF INFLUENCE OF HC1 ON HYDROLYTIC COPOLYCONDENSATION OF BIFUNCTIONAL ORGANOCHLOROSILANES WITH TRIMETHYLCHLOROSILANE" JOURNAL OF GENERAL CHEMISTRY USSR, CONSULTANTS BUREAU. NEW YORK, US, Bd. 61, Nr. 6, 1. Juni 1991 (1991-06-01), Seiten 1257-1261, XP000274401

## Beschreibung

Niedermolekulare Siloxane sind seit langer Zeit als verdampfbare Träger in kosmetischen Zubereitungen bekannt (T. Koini, M. D. Berthiaume, A. Huber in: SÖFW-Journal, 125. Jahrgang 4/99, 22.). Insbesondere Octamethylcyclotrasiloxan war durch seine relativ hohe Verdampfungsgeschwindigkeit und sein angenehmes Hautgefühl verbreitet in kosmetischen Anwendungen zu finden (Product Description SF 1173, SF 1202, SF 1204, SF 1246 General Electric (GE) Silicones CDS 4958-E/ENG/0797).

In jüngster Zeit wurden gegenüber der Verwendung von Octamethylcyclotetrasiloxan in kosmetischen Formulierungen unter Gesichtspunkten physiologischer Verträglichkeit Bedenken geäußert. Im Tierversuch beobachtete man bei extremen Dosierungen eine verminderte Fertilität beobachtet. Es wurden daher in letzter Zeit vermehrt Anstrengungen unternommen, Octamethylcyclotetrasiloxan in kosmetischen Formulierungen durch geeignete Komponenten zu ersetzen, wobei diese Ersatzstoffe das Leistungs- und Eigenschaftsprofil von Octamethylcyclotetrasiloxan bisher nicht erreichen. So ist zum Einen die Verdampfungsgeschwindigkeit von dem als Ersatzstoff für Octamethylcyclotetrasiloxan verwendeten Decamethylcyclopentasiloxan deutlich niedriger. Zum Anderen vermitteln sowohl das lineare Octametyltrisiloxan als auch Decamethyltetrasiloxan nicht das für die cyclischen Siloxane typische seidige, nicht - ölige Hautgefühl.

Es ist bisher nicht gelungen, verdampfbare Trägersubstanzen zu entwickeln, die genügend flüchtig sind und gleichzeitig das gute Hautgefühl der cyclischen Siloxane zeigen und es so ermöglichen, bei der Herstellung von kosmetischen Zubereitungen auf die Verwendung von Octamethylcyclotetrasiloxan (=D4) als verdampfbarem Träger zu verzichten. Unter verdampfbaren Trägern sollen hier Siloxane verstanden werden, die einen Siedepunkt bei Normaldruck von unter 250 °C und ein Verdampfungsverhalten gemessen nach DIN 53249 ähnlich wie D4 haben.

JP-A2-2000 204 398 (133:106626CA) Offenbart Trisiloxane enthaltende Reinigungsmittel.

JP-A2 55-020 737 (93:137891 CA) Offenbart die Verwendung von Disiloxanen in Kosmetischen Formulierungen.

KOPYLOV et al., Journal of General Chemistry USSR, 61 (1991), Nr. 6, Teil 2, Seiten 1257-1261 Offenbart ein Verfahren der Copolykondensation von bifunktionellen Organochlorsilanen mit Trimethylchlorsilan.

In EP 0 980 885 sind Mischungen flüchtiger Siloxane beschrieben, die in kosmetischen Formulierungen eingesetzt werden können. Die Aufgabenstellung der EP 980 855. ist dabei, dass flüchtige lineare Siloxane gesucht wurden, die nach 30 Minuten gemäß DIN 53249 erst zu 80-85 Gew.% verdampft sind.

Diese Siloxane sollen somit langsamer als Octamethylcyclotetrasiloxan (=D4) verdampfen.

WO 98-32418 beschreibt transparente Antiperspiranzgele, die u.a. die verdampfbaren Gemische der linearen Siloxane von EP 980 855 sowie zusätzlich Cyclosiloxane enthalten können.

Es findet sich aber kein Hinweis, daB Alkylsiloxane mit einer zum D4 vergleichbaren Flüchtigkeit bevorzugt sind oder daß solche dort als Ersatz für D4 erkannt wurden.

Im Gegensatz zu EP 980 855 u. WO 98-32418 war es Gegenstand dieser Erfindung, reine Substanzen zu entwickeln, die insbesondere in kosmetischen Formulierungen aber auch in anderen Reingungsformulierungen als flüchtige Trägersubstanz an Stelle von D4 eingesetzt werden können, wobei das Verdampfungsverhalten dem des Octamethylyclotetrasiloxans möglichst nahekommen, über große Temperaturbereiche flüssig bleiben und deren Stockpunkt insbesondere bei tieferen Temperaturen liegen soll. Dabei ist es vorteilhaft, dass durch die Verwendung von reinen Einzelsubstanzen die Anzahl der in kosmetischen Formulierungen enthaltenen Substanzen reduziert werden kann. Hierdurch minimiert man Wechselwirkungen der verschiedenen Stoffe untereinander, so daß die Produktsicherheit und Qualitätssicherung entscheidend verbessert werden. D.h. die bevorzugten Formulierungen sind die Formulierungen, in denen das Octamethylcyclotetrasiloxan bzw. lineare permethylierte Siloxane mit vergleichbarem Verdampfungsverhalten ausschließlich durch die erfindungsgemäßen linearen Alkylmethylsiloxane in reiner Form mit einem Gehalt oberhalb 85% ersetzt werden.

Die erfindungsgemäßen verdampfbaren Siloxane weisen darüberhinaus kein bzw. geringes 'Whitening' auf. Darunter versteht man das in US 5,922,309 beschriebene Phänomen weißer Rückstände auf der Haut oder dem Textil.

Ziel der Erfindung war es darüberhinaus, Siloxane mit seidigem, nicht-öligem Hautgefühl und mit Verdampfungseigschaften zu entwickeln, die denen des Octamethylcyclotetrasiloxans ähnlich sind bzw. dieses übertreffen. Octamethylcyclotetrasiloxan hat mit 4 °C einen Stockpunkt bei relativ hoher Temperatur was bei Transport, Lagerung und Verarbeitung von Formulierungen sowie Rohstoff je nach klimatischen Verhältnissen zu Problemen führen kann. Ziel dieser Erfindung war es deshalb auch, Siloxane mit günstigerem, tieferem Stockpunkt zu entwickeln. Daneben war es Ziel der Erfindung, Siloxane mit einer guten Kompatibilität mit kosmetischen Rohstoffen zu entwickeln.

Überraschenderweise wurde gefunden, dass ausgewählte niedermolekulare lineare, mit kurzkettigen Alkylgruppen modifizierte Siloxane gute, durch die Wahl der Alkylgruppen steuerbare Verdampfungseigenschaften besitzen und gleichzeitig ein den cyclischen Siloxanen entsprechendes seidiges, nicht-öliges Hautgefühl aufweisen.

Bei den erfindungsgemäß verwendeten Siloxanen handelt es sich um die folgenden Verbindungen:
1,1,1,3,5,5,5-Heptamethyl-3-ethyltrisiloxan, 1,1,1,5,5,5-Hexamethyl-3,3-diethyltrisiloxan, 1,1,3,3,5,5-Hexamethyl-1,5-diethyltrisiloxan, 1,1,1,3,5,5,5-Heptamethyl-3-propyltrisiloxan, 1,1,1,5,5,5-Hexamethyl-3,3-dipropyltrisiloxan oder 1,1,1,3,5,5,5-Heptamethyl-3-butyltisiloxan unter der Massgabe, dass es sich bei den Propyl- und Butylgruppen um lineare oder verzweigte Substituenten handeln kann als verdampfbare Träger in kosmetischen Formulierungen .

Ein verdampfbarer Träger im Sinne der Erfindung meint eine Substanz mit einem geeigneten Verdampfungsverhalten, die in eine kosmetische bzw. Reinigungsformulierung eingebracht wird. Dieser Träger ermöglicht das Einarbeiten verschiedener Komponenten in diese Formulierung ohne Entmischung. Der Träger muss auf der einen Seite eine gewisse Flüchtigkeit aufweisen, auf der anderen Seite aber auch eine gute Applikation des Gesamtproduktes gewährleisten.
Bevorzugt ist 1,1,1,3,5,5,5-Heptamethyl-3-propyltrisiloxan (CAS-Nr. 29054-80-6) 1,1,1,5,5,5-Hexamethyl-3,3-diethyltrisiloxan, sowie 1,1.1,3,5,5,5-Heptamethyl-3-butyltrisiloxan (CAS-Nr. 18138-63-1). Das am meisten bevorzugte Siloxan ist 1,1,1,3,5,5,5-Heptamethyl-3-ethyltrisiloxan (CAS-Nr. 17861-60-8).

Die erfindungsgemäß verwendeten Verbindungen sind an sich bekannt (W. Noll, Chemie und Technologie der Silicone, 2. Auflage, Verlag Chemie Weinheim 1968, S. 216 f.). In bevorzugter Weise lassen sie sich durch Addition gezielt ausgewählter Alkene an geeignete SiH funktionelle Siloxane und gegebenenfalls anschließende Äquilibrierungsreaktionen herstellen. Eine alternative Herstellung ist auch durch die Einführung des Alkylsubstituenten in Chlorsilane über metallorganische Reagentien (z.B.GRIGNARD- bzw. WURTZ-Reagenzien) mit anschliessender Hydrolyse des nach dieser Reaktion erzeugten Alkylchorsilanes, bevorzugt Methylalklydichlorsilane, und die Äquilibrierung mit den Hydrolyseprodukten anderer Methylchlorsilane.

Die erfindungsgemäß verwendeten Siloxane zeichnen sich durch Stockpunkte, die weit unter dem Gefrierpunkt von Wasser liegen, aus, was sowohl den Transport als auch die Einarbeitung und Verwendung in kosmetische Formulierungen erleichtert.

Die erfindungsgemäß verwendeten Siloxane wurden in kosmetische Formulierungen eingearbeitet und diese dann von unabhängigen Testpersonen in ihrem Hautgefühl bewertet. Dabei wurde das Hautgefühl als besser bewertet als bei der Verwendung von Decamethylcyclopentasiloxan. Zu der Verwendung von Octamethylcyclotetrasiloxan konnten hingegen keine signifikanten Unterschiede beobachtet werden.
In einer bevorzugten Verfahrensweise können die erfindungsgemäß verwendeten Verbindungen durch Destillation eines Gemisches aus Hexamethyldisiloxan, 1,1,1,3,5,5,5-Heptamethyl-3-ethyltrisiloxan sowie oligomeren Verbindungen, von denen das folgende Homologe (1,1,1,3,5,7,7,7-Octamethyl-3,5-diethyltetrasiloxan) einen Siedepunkt von > 220°C aufweist, erhalten werden. Die für diese Reaktion eingesetzten Silane fallen als Nebenprodukte bei der Direktsynthese von Dimmethyldichlorsilan an (H.-H. Moretto, M. Schulze, G. Wagner in: Ullmann's Encyclopedia of Industrial Chemistry, Sixth Edition 1998, Wiley-VCH, Weinheim) Durch Cohydrolyse von Methylethyldichlorsilan mit Trimethylchlorsilan entstehen z.B. geeignete Gemische, die nach Destillation zu 1,1,1,3,5,5,5-Heptamethyl-3-ethyltrisiloxan aufbereitet werden können. Überraschend wurde gefunden, dass bei dieser Verfahrensvariante, ausgehend von einer komplexen Mischung von Nebenprodukten nach Cohydrolyse durch Äquilibrierung und einfache Destillation die erfindungsgemäß verwendeten Produkte in hochreiner definierter Form zugänglich sind.

Alternativ können Mischungen aus 1,1,1,3,5,5,5-Heptamethyl-3-ethyltrisiloxan sowie oligomeren Verbindungen aus einem Hydrolysat des Methylethyldichlorsilans und dessen anschließender sauer katalysierter Äquilibrierung mit Hexamethyldisiloxan erhalten werden (G. Koerner in: Silicone-Chemie und Technologie, Symposium, Vulkan-Verlag Essen 1998, Seite 3).

Es ist ebenfalls bevorzugt, die erfindungsgemäßen Siloxane durch metallkatalysierte Addition (Hydrosilylierung) von 1-Alkenen an trimethylsiloxyterminiertes Methylhydrogenpolysiloxan oder cyclische Methylhydrogensiloxane über anschließende sauer katalysierte Äquilibrierung mit Hexamethyldisiloxan zu erhalten (Bogdan Marciniec (Hrsg.) Comprehensive Handbook an Hydrosilylation, Pergamon Press, Oxford. 1992, S. 99 ff.). Geeignete Metalle sind Ni, Rh. Ru, oder Pt , bevorzugt Pt, in Form der kolloidalen Metalle selbst, deren Salze oder Komplexe mit der formalen Oxidationsstufe 0 bis VI.

Die Erfindung betrifft weiterhin die Verwendung mindestens eines der oben genannten Siloxane in Zusammensetzungen, die aus der Gruppe ausgewählt werden, die einschließt: kosmetische Zubereitungen, Hautpflegeprodukte, Sonnenschutzprodukte, Haarpflegeprodukte, Deodorantien, Antiperspirantien, Produkte der dekorativen Kosmetik, Lippenstifte, Mundpflegeprodukte und Nagelpflegeprodukte. In den vorstehend genannten Anwendungen bzw. Produktzusammensetzungen können die erfindungsgemäß verwendeten Siloxane zweckmäßig in Mengen von 0,01 bis 99 Gew.-% bezogen auf das Gesamtgewicht der Formulierung bzw. der Produktzusammensetzung verwendet werden. Bevorzugt ist eine Verwendung in Mengen von 0,5 bis 70 Gew.-%, besonderes bevorzugt in Mengen von 1,0 bis 60 Gew.-%.

Bevorzugt werden die Siloxane in Mengen von 0,01 % bis 99 % in kosmetischen Zubereitungen, wie zum Beispiel Deodorantien, Antiperspirantien, dekorativer Kosmetik, Cremes, Lotions, Hautpflegeoelen, Shampoos, Conditioners, Sprays, Aerosolen, Pudern, Suncare-Produkten, Gelen oder Pasten u.a als verdampfbarer Träger eingesetzt.
Insbesondere ist bevorzugt, keinerlei weitere Cyclosiloxane in diesen Formulierungen einzusetzen.
Die erfindungsgemäß verwendeten Siloxane zeigen eine ausgeprägte Kompatibilität mit gängigen kosmetischen Rohstoffen. Sie mischen sich sowohl mit Pflanzenölen wie Sonnenblumen-, Avocado- oder Jojobaöl als auch mit Estern, wie Isopropylmyristat, Ethylhexylpalmitat oder Isononylisononanoat. Darüber hinaus sind die erfindungsgemäßen Siloxane löslich in Alkoholen, wie Ethanol und Isopropanol, sowie den nach INCI gelisteten nicht-cyclischen Siloxanen, wie Dimethicone, Phenyl Trimethicone oder Bis-Phenylpropyl-Dimethicone.

Die nachfolgenden Beispiele sollen die Verwendung der erfindungsgemäßen Substanzen illustrieren, ohne deren Verwendungmöglichkeit als bestmöglichen D4-Ersatz zu limitieren.

### Beispiele:

### Beispiel 1:

### Herstellung von 1,1,1,3,5,5,5-Heptamethyl-3-ethyltrisiloxan

In einem mit Wasser gekühlten Kolben ausgerüstet mit Bodenablass, Rührer, Thermometer, Rückflusskühler und Tropftrichter werden 3900 g Wasser vorgelegt und unter Rühren 1430 g (10 mol) Methylethyldichlorsilan zudosiert. Durch Regelung der Dosiergeschwindigkeit wird die Temperatur auf maximal 40°C begrenzt. Nach beendeter Dosierung wird der Rührer abgestellt und nach Ausbildung einer klaren Phasentrennung die untere, wässrige, salzsaure Phase abgetrennt und verworfen. Die obere Ölphase wird einmal mit Wasser gewaschen und anschließend in einen Kolben, ausgerüstet mit Rührer, Thermometer, Wasserabscheider und Rückflusskühler überführt. Zu der Ölphase werden 3240 g (20 mol) Hexamethyldisiloxan und 40g (0,41 mol) 98 - 100%ige Schwefelsäure gegeben. Das Gemisch wird zum Kondensieren der Silanol-Gruppen sowie zur Entfernung restlichen Wassers auf Rückflusstemperatur aufgeheizt. Nach zwei Stunden Rückfluss werden 10 g (0,047 mol) Perfluorbutansulfonsäure zugegeben und die Reaktionsmischung weitere 4 h bei 100°C gerührt. Nach Abkühlen auf unter 70°C werden zur Neutralisation 80 g Soda und 15 g Wasser und 3 g 25%ige Ammoniaklösung zugegeben, eine Stunde nachgerührt und nach Überprüfung auf Säurefreiheit erneut auf Rückflusstemperatur aufgeheizt. Nach einer Stunde Rückfluss wird abgekühlt und filtriert. Man erhält 3820 g eines Rohproduktes, bestehend aus 58% Hexamethyldisiloxan, 23% 1,1,1,3,5,5,5-Heptamethyl-3-ethyltrisiloxan und oligomeren Siloxanen, welches durch fraktionierte Destillation 717 g 98%-iges 1,1,1,3,5,5,5-Heptamethyl-3-ethyltrisiloxans mit einer Siedetemperatur von 169-172 °C liefert. Sumpf und abgetrenntes Hexamethylsiloxan können weiteren Äquilibrierungen zugeführt werden.

### Beispiel 2

### Herstellung von 1,1,1,3,5,5,5-Heptamethyl-3-propyltrisiloxan

In einen beheizbaren 41 Emailautoklaven, ausgerüstet mit Rührer und Thermometer, werden 2000 g (= 30 mol SiH) eines, bei der technischen Herstellung linearer trimethylsiloxyterminierter Methylhydrogenpolysiloxane angefallenen Destillates, bestehend aus 75% cyclischen Methylhydrogensiloxanen mit einer mittleren Ringgröße von 4,8 D^{H} Einheiten und 25% Trimethylsiloxy-terminierten Methylhydrogensiloxanen mit einer mittleren Kettenlänge von 3 (=MeSiHO)-Einheiten, vorgelegt und mit Stickstoff überlagert. Der Druck des Autoklaven wird mit einer Vakkumpumpe auf 100 mbar reduziert, der Autoklaveninhalt wird auf 65°C aufgeheizt, 1g eines 2% Platin enthaltenden kommerziellen Pt° Octanal/Octanol Komplexes (Gelest) werden mit 20 ml Hexamethyldisiloxan verdünnt in den Autoklaven eingesaugt und der Druck des Autoklaven erneut auf 100 mbar abgesenkt Anschließend wird Propen aus einer Stahlflasche so eingeleitet, daß der Druck 2 bar und die Temperatur, durch die nahezu sofort erkennbare exotherme Reaktion, 130°C nicht übersteigt. Nach Abklingen der Exothermie wird noch 1h bei 130°C und 2 bar Propen Druck nachgerührt, der Autoklav entspannt, und entgast. Man erhält 3300g Addukt mit einem SiH Gehalt von < 0,01 mmol/g.

In einem Kolben ausgerüstet mit Rührer, Thermometer, Wasserabscheider, Rückflußkühler und Stickstoffüberlagerung werden 658g (6 mol D^{Prop} (=PropylMeSiO)ₙ) des auf diese Weise erhaltenen Produktes und 1458 g (9 mol) Hexamethyldisiloxans vorgelegt mit 102 g Bleicherde Tonsil Standard 310 FF (Südchemie) versetzt und 6 h bei einer Sumpftemperatur von ca. 100°C am Wasserabscheider gerührt, abgekühlt und filtriert. Man erhält 2100g eines Rohproduktes bestehend aus 62% Hexamethyldisiloxan, 14% 1,1,1,3,5,5,5-Heptamethyl-3-propyltrisiloxan und 24% oligomeren Siloxanen, welches durch fraktionierte Destillation 410 g 98%iges 1,1,1,3,5,5,5-Heptamethyl-3-propyltrisiloxan mit einer Siedetemperatur von 192 - 194°C liefert. Sumpf und abgetrenntes Hexamethyldisiloxan können weiteren Äquilibrierungen zugeführt werden.

### Referenz-

### Beispiel 3

### Herstellung von 1,1,1,3,3-Pentamethyl-3-butyldisiloxan

In einen beheizbaren 4 1 Emailautoklaven, ausgerüstet mit Rührer und Thermometer werden 2010 g (= 30 mol SiH) 1,1,3,3-Tetramethyldisiloxan unter Stickstoffüberlagerung vorgelegt. Der Druck des Autokalven wird mit einer Vakumpumpe auf 250 mbar reduziert, der Autoklaveninhalt wird auf 65°C aufgeheizt, 1g eines 2% Platin enthaltenden kommerziellen Pt°-Octanal/Octanol Komplexes (Gelest) werden mit 20 ml Hexamethyldisiloxan verdünnt in den Autoklaven eingesaugt. Anschließend wird 1-Buten aus einer Stahlflasche so eingeleitet, daß der Druck 2 bar und die Temperatur, durch die nahezu sofort erkennbare exotherme Reaktion, 130°C nicht übersteigt. Nach Abklingen der Exothermie wird das gebildete 2-Buten bis 100 mbar abgezogen, anschließend erneut 1-Buten eingeleitet und noch 1 h bei 130°C und 2 bar Buten Druck nachgerührt, der Autoklav entspannt, und bis 100 mbar entgast. Man erhält 3500g Addukt mit einem SiH Gehalt von < 0,01 mmol/g.
In einen Kolben ausgerüstet mit Rührer, Thermometer, Wasserabscheider, Rückflußkühler und Stickstoffüberlagerung werden 984g Addukt (8 mol M^{Butyl} (=Butyl-Me₂SiO_{0.5}) und 1312 (8 mol) Hexamethyldisiloxans vorgelegt, mit 0,1% Schwefelsäuren und 0,05% Perfluorbutansulfonsäure versetzt, 4h bei einer Sumpftemperatur von 70°C, mit 20g calzinierter Soda, 3g Wasser und 0,5g 25%iger NH₃-Lsg. neutralisiert, bis 110°C andestilliert, abgekühlt und filtriert. Man erhält 1950g eines Rohproduktes bestehend aus 36% Hexamethyldisiloxan, 47% 1,1,1,3,3-Pentamethyl-3-butyldisiloxan und 17% 1,1,3,3-Tetramethyl-1,3-dibutyl-disiloxan, welches durch fraktionierte Destillation 810 g 97%iges 1,1,1,3,3-Pentamethyl-3-butyldisiloxan mit einer Siedetemperatur von 166 - 169°C liefert. Sumpf und abgetrenntes Hexamethyldisiloxan können weiteren Äquilibrierungen zugeführt werden.

### Beispiel 4

### Verdampfungsverhalten

In der nachfolgenden Tabelle 1 und Abbildung 1 ist das in Anlehnung an DIN 53249 gemessene Verdampfungsverhalten von Octamethylcyclotetrasiloxan, Decamethylcyclopentasiloxan und der Beispielverbindungen 1 und 3 als nicht verdampften Rückstand in [%] wiedergegeben.

**Tab. 1 Verdampfungsverhalten nach DIN 53249 gemessen als verbleibenden Siloxan-Rückstand**

| **Zeit [min]** | **Octamethylcyclo tetrasiloxan [%]** | **Decamethylcyclo pentasiloxan [%]** | **Beispiel 1 [%]** | **Beispiel 3 [%]** |
|---|---|---|---|---|
| 0 | 100 | 100 | 100 | 100 |
| 5 | 68.3 | | 59.7 | |
| 10 | 37.4 | | 23.9 | 82 |
| 15 | 11.4 | | 2.8 | |
| 20 | 0 | | 0.6 | 63 |
| 25 | 0 | | 0.1 | |
| 30 | 0 | 70.2 | 0.1 | 43 |
| 35 | 0 | | 0.1 | |
| 40 | | | | 25 |
| 50 | | | | 11 |
| 60 | | 39.6 | | 3 |
| 70 | | | | 0 |
| 90 | | 14.5 | | |
| 120 | | 1.2 | | |
| 150 | | 0 | | |

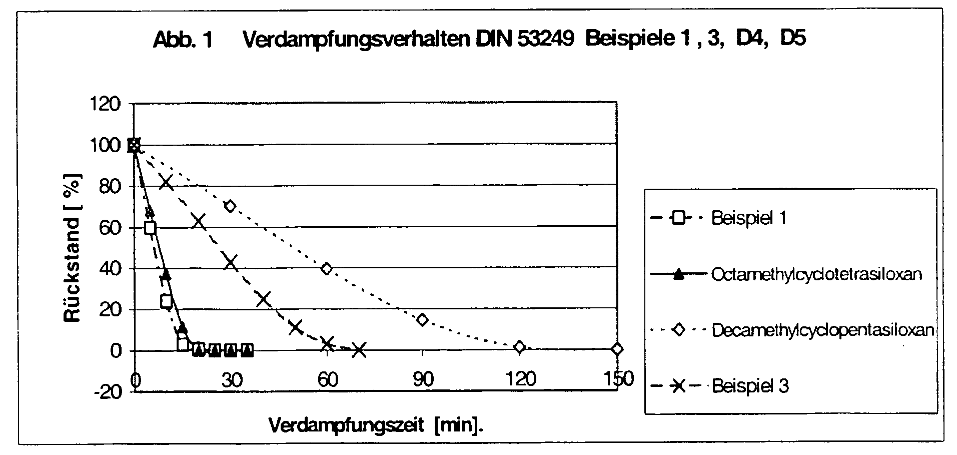

Im Vergleich zu Octamethylcyclotetrasiloxan verdampft Decamethylcyclopentasiloxan deutlich langsamer, so dass bei der Applikation von Decamethylcyclopentasiloxanenthaltenden Formulierungen ein öliges, nasses Gefühl auf der Haut auftritt. Dieses bei der Verwendung der Beispielverbindung 1 wird vermieden, die ein zu Octamethylcyclotetrasiloxan analoges Verdampfungsverhalten zeigt. Darüber hinaus vermitteln Formulierungen, die die Beispielverbindung 1 enthalten, ein sehr angenehmes seidiges, nicht-öliges und leichtes Hautgefühl.
Die Flüchtigkeit der Beispielverbindung 3 liegt zwischen der von Oetamethylcyclotetrasiloxan und Decamethylcyclopentasiloxan und kann daher verwendet werden, wenn eine Flüchtigkeit verlangt wird, die zwischen der von Octamethylcyclotetrasiloxan und Decamethylcyclopentasiloxan liegen soll. Formulierungen die Beispielverbindung 3 enthalten, weisen ein substantielleres und etwas öligeres Hautgefühl auf.
Durch Abmischungen der erfindungsgemässen Siloxane ist es möglich, eine bestimmte zu Octamethylcyclotetrasiloxan abgestufte, angestrebte Flüchtigkeit oder aber ein bestimmtes wünschenswertes, von D4 abweichendes Hautgefühl gezielt einzustellen.

### Beispiel 5

### Herstellung eines kosmetischen Sprays

Die typische Hilfsstoffe in derartigen Zusammensetzungen sind z.B. diejenigen Stoffe, die in A. Domsch: Die kosmetischen Präparate Bd. I u. II 4. Aufl. Verl. für chem. Industrie, H. Ziolkowsky KG , Augsburg sowie im International Cosmetic Ingredient Dictionary and Handbook 7^{th} Ed. 1997 by J.A. Wenniger, G.N. McEwen Vol. 1-4 by The Cosmetic, Toiletry and Fragrance Association Washington DC beschrieben.
Phase 1: PEG-8 Bienenwachs (6.0 %), Beispielverbindung 1 (5.5 %), Jojobaöl (5.5 %), Cocoglyceride (5.0 %), Oleyl Erucat (4.0 %).
Phase 2: Wasser (78.6 %), Guar Hydroxypropyltrimonium chloride (0.3 %), Xanthan Gurn (0.1 %), Sodium Lactate (2.0 %), Phenonip (0.4 %).

Beide Phasen werden getrennt voneinander auf 65 °C erhitzt Dann wird unter intensivem Rühren die heiße Phase 1 zu der heißen Phase 2 gegeben und 10 Minuten bei 60 °C gerührt. Das Abkühlen erfolgt bei reduzierter Rührgeschwindigkeit.

### Beispiel 6

### Herstellung eines Foundation Make-up

Phase 1: Wasser (q.s.), Xanthan Gum (0.3 %), Propylenglykol (5.0 %), Triethanolamin (1.4%).
Phase 2: Glycerylstearat SE (3.9 %), Isononyl Isononanoat (3.0 %), Siliconöl SF 1555® GE Silicones (8.5 %), Beispielverbindung 2 (3 %), Dimethicone SF 1236® GE Silicones (1.0 %), Stearinsäure (3.9 %).
Phase 2: Glycerylstearat SE (3.9 %), Isononyl Isononanoat (3.0 %), Bis-Phenylpropyl Dimethicone (SF 1555® GE Silicones) (8.5 %), Beispielverbindung 2 (3 %), Dimethicone (5000 cStk) SF 1236® GE Silicones (gum) (1.0 %), Stearinsäure (3.9 %).
Phase 3: Pigment Blend (3.3 %).
Phase 4: Konservierungsmittel (q.s.).

Herstellung: Unter intensivem Rühren wird der Xanthan Gum in Waser dispergiert. Die restlichen Bestandteile von Phase 1 werden zugegeben und auf 75 bis 77 °C erhitzt. Die Bestandteile für Phase 2 werden ebenfalls unter intensivem Rühren gemischt und auf 75 bis 77 °C erhitzt. Dann wird Phase 2 zu Phase 1 hinzugegeben und 5 Minuten bei mindestens 70 °C homogenisiert. Anschließend wird Phase 3 hinzugefügt, erneut homogenisiert und auf 50 °C abgekühlt. Schließlich wird das Konservierungsmittel zugegeben und unter langsamen Rühren auf 25 °C abgekühlt.

### Beispiel 7

### Herstellung einer Creme Foundation

Phase 1: C30-45 Alkyl Dimethicone (SF 1642® GE Silicones) (11.0 %), Cetearyl Methicone (SF 1632® GE Silicones) (4.0 %), Stearinsäure (11.5 %), Beispielverbindung 3 (4.0 %), Lanolin (3.0 %),
Phase 2: Wasser (q.s.), Propylenglycol (3.4 %), Triethanolamin (2.9 %), Konservierungmittel (q.s.).
Phase 3: Polymethylsilsesquioxane (Tospearl 145A® GE Silicones) (10 %), Pigment Blend (18 %).

Herstellung: Phase 1 und Phase 2 werden separat auf 70 °C erhitzt. Dann wird Phase 2 zu Phase 1 unter Rühren hinzugefügt, bis die Mischung abgekühlt ist. Unter langsamem Rühren wird dann diese Mischung zu Phase 3 gegeben.

## Patentansprüche

1. Verwendung von 1,1,1,5,5,5-Hexamethyl-3,3-diethyltrisiloxan, 1,1,1,3,5,5,5-Heptamethyl-3-ethyltrisiloxan, 1,1,3,3,5,5-Hexamethyl-1,5-diethyltrisiloxan, 1,1,1,3,5,5,5-Heptamethyl-3-propyltrisiloxan, 1,1,1,5,5,5-Hexamethyl-3,3-dipropyltrisiloxan oder 1,1,1,3,5,5,5-Heptamethyl-3-butyltrisiloxan als Einzelverbindung oder in Mischung unter der Massgabe, dass es sich bei den Propyl- und Butylgruppen um lineare oder verzweigte Substituenten handeln kann als verdampfbarer Träger in kosmetischen Formulierungen.

2. Verwendung von 1,1,1,3,5,5,5-Heptamethyl-3-ethyltrisiloxan gemäß Anspruch 1.

3. Verwendung von 1,1,1,3,5,5,5-Heptamethyl-3-propyltrisiloxan gemäß Anspruch 1.

4. Verwendung von 1,1,1,3,5,5,5-Heptamethyl-3-butyltrisiloxan gemäß Anspruch 1.

5. Verwendung mindestens eines Siloxanes nach Anspruch 1 in Zusammensetzungen, die aus der Gruppe ausgewählt werden, die einschließt: kosmetische Zubereitungen, Hautpflegeprodukte, Sonnenschutzprodukte, Haarpflegeprodukte, Deodorantien, Antiperspirantien, Produkte der dekorativen Kosmetik, Lippenstifte, Mundpflegeprodukte sowie Nagelpflegeprodukte.

## Claims

1. Use of 1,1,1,5,5,5-hexamethyl-3,3-diethyltrisiloxane, 1,1,1,3,5,5,5-heptamethyl-3-ethyltrisiloxane, 1,1,3,3,5,5-hexamethyl-1,5-diethyltrisiloxane, 1,1,1,3,5,5,5-heptamethyl-3-propyltrisiloxane, 1,1,1,5,5,5-hexamethyl-3,3-dipropyltrisiloxane or 1,1,1,3,5,5,5-heptamethyl-3-butyltrisiloxane as individual compound or in a mixture, with the proviso that the propyl and butyl groups may be linear or branched substituents as vaporizable carrier in cosmetic formulations.

2. Use of 1,1,1,3,5,5,5-heptamethyl-3-ethyltrisiloxane according to Claim 1.

3. Use of 1,1,1,3,5,5,5-heptamethyl-3-propyltrisiloxane according to Claim 1.

4. Use of 1,1,1,3,5,5,5-heptamethyl-3-butyltrisiloxane according to Claim 1.

5. Use of at least one siloxane according to Claim 1 in compositions which are chosen from the group which includes: cosmetic preparations, skincare products, sunscreen products, hair care products, deodorants, antiperspirants, products of decorative cosmetics, lipsticks, mouth care products and nail care products.

## Revendications

1. Utilisation du 1,1,1,5,5,5-hexaméthyl-3,3-diéthyltrisiloxane, du 1,1,1,3,5,5,5-heptaméthyl-3-éthyltrisiloxane, du 1,1,3,3,5,5-hexaméthyl-1,5-diéthyltrisiloxane, du 1,1,1,3,5,5,5-heptaméthyl-3-propyltrisiloxane, du 1,1,1,5,5,5-hexaméthyl-3,3-dipropyltrisiloxane ou du 1,1,1,3,5,5,5-heptaméthyl-3-butyltrisiloxane sous forme de composé unique ou en mélange avec la condition que les groupes propyle et butyle puissent être des substituants linéaires ou ramifiés comme support évaporable dans des formulations cosmétiques.

2. Utilisation du 1,1,1,3,5,5,5-heptaméthyl-3-éthyltrisiloxane selon la revendication 1.

3. Utilisation du 1,1,1,3,5,5,5-heptaméthyl-3-propyltrisiloxane selon la revendication 1.

4. Utilisation du 1,1,1,3,5,5,5-heptaméthyl-3-butyltrisiloxane selon la revendication 1.

5. Utilisation d'au moins un siloxane selon la revendication 1 dans des compositions qui sont choisies dans le groupe qui inclut : les préparations cosmétiques, les produits de soins pour la peau, les produits de protection contre le soleil, les produits de soins pour les cheveux, les désodorisants, les antisudoraux, les produits de cosmétique décorative, les bâtons de rouge à lèvres, les produits de soins buccaux ainsi que les produits de soins pour les ongles.
